# EUROPÄISCHE PATENTSCHRIFT

(11) **EP 2 085 071 B1**
(45) Veröffentlichungstag und Bekanntmachung des Hinweises auf die Patenterteilung: **02.12.2015**
(21) Anmeldenummer: 09000651.1
(22) Anmeldetag: 19.01.2009
(51) Int. Cl.: A61K 47/10, A61K 47/12, A61K 47/18, A61K 9/08, A61K 9/10, A61K 31/137

(54) **Oral oder nasal applizierbare Epinephrin-haltige Zubereitungen mit verbesserten Eigenschaften**
Orally or nasally administrable preparations containing epinephrine with improved characteristics
Préparations orales ou nasales contenant de l'épinéphrine applicable ayant des propriétés améliorées

(30) Priorität: 01.02.2008 DE 102008007198
(43) Veröffentlichungstag der Anmeldung: 05.08.2009
(73) Patentinhaber: Infectopharm Arzneimittel und Consilium GmbH, 64646 Heppenheim (DE)
(72) Erfinder: Zöller, Manfred, Dr., 69469 Weinheim (DE); Pelzer, Kris, 69469 Weinheim (DE); Wachall, Bertil, Dr., 69502 Hemsbach (DE)
(74) Vertreter: Müller, Claudia

(56) Entgegenhaltungen:
- EP-A1- 1 586 347
- WO-A-01/82931
- WO-A-99/38492
- WO-A-2004/054556
- WO-A2-01/91848

## Beschreibung

### Gegenstand der Erfindung

Die vorliegende Erfindung betrifft Epinephrin-haltige Zubereitungen mit verbesserten Eigenschaften, insbesondere verbesserter Stabilität. Dies wird vor allem durch eine gezielte Auswahl bestimmter Stabilisatoren, nämlich Zitronensäure bzw. Zitronensäuresalz und / oder Ethylendiamintetraessigsäure oder ein Salz hiervon in Kombination mit Ethanol erreicht. Überraschender Weise können auf diese Weise stabile wässrige (vor allem treibgasfreie) Zubereitungen zur nasalen oder oralen Anwendung in den Atemwegen erhalten werden, wobei diese darüber hinaus, insbesondere beim Transport, auch nicht mehr bzw. nicht ständig, gekühlt werden müssen. Dies führt zu einer erheblich verbesserten Verfügbarkeit und damit Anwendersicherheit.

### Stand der Technik

Epinephrin (Adrenalin)- haltige Zubereitungen oder Mittel, enthaltend pharmazeutisch verträgliche Derivate wie insbesondere Salze hiervon, werden vor allem zur Behandlung einer akuten oder chronischen allergischen Reaktion wie allergischer Schock, allergiebedingter Atemnot, Pseudokrupp, Säuglingsbronchiolitis bzw. obstruktive Bronchitis des Säuglings und Kleinkindes, Asthma, sonstige Formen der Atemnot oder auch Herz-Kreislauf-Stillstand eingesetzt. Zur Behebung derartiger Störungen kann der Wirkstoff sowohl parenteral verabreicht werden, in der Mundhöhle oder im Rachenraum appliziert werden als auch oral bzw. nasal inhaliert oder instilliert werden. Je nach Applikationsform werden jedoch unterschiedliche Anforderungen an die Zubereitung gestellt, da der Wirkstoff einer raschen Zersetzung sowohl durch Temperatur als auch durch Licht und Milieubedingen wie Sauerstoff, pH-Wert, und Kontakt zu verschiedenen Metallen unterliegt. Dies beruht darauf, dass Adrenalin als Phenylethanolamin ein hohes Reduktionspotential insbesondere in Lösungen, vor allem in wässrigen Lösungen aufweist und neben dem oxidativen Abbau auch der Racemisierung unterliegt. Letztendlich führen die genannten Faktoren zur Zersetzung der aktiven Substanz in Melanin- Abbauprodukte, welche sich auch an der dunklen Verfärbung von Epinephrin-haltigen Lösungen zeigt. Wesentliche Verbesserungen der Haltbarkeit Epinephrin-haltiger Zubereitungen können daher schon durch Eliminierung oxidationsfördernder Faktoren wie z. B. Licht, Schwermetallionen oder Temperatur erfolgen. Des Weiteren können Stabilisatoren wie Ascorbinsäure, Aminosäuren, oder insbesondere auch Sulfite wie Natriumdisulfit, hinzugesetzt werden. Untersuchungen zur Stabilität Epineph-rinhaltiger Zubereitungen sind im Kommentar zu Ph. Eu. 4.00/0254; ferner in K. Thoma, M. Struwe in Pharm. Acta Helv. V. 61, Nr. 1, 1986, Seite 2-9; K. Thoma, M. Struwe in Pharm. Acta Helv. V. 61, Nr. 1, 1986, Seite 34-41; sowie H. Wollmann, G. Raether in Pharmazie 38 (1982), H1, Seite 37-42 beschrieben. Verschiedene Testreihen mit unterschiedlichen Säuren, Aminosäuren etc. führten zu jeweils unterschiedlichen Ergebnissen. Insbesondere Ascorbinsäure, N-Acetylcystein sowie vor allem die oben genannten Sulfite werden als geeignete Stabilisatoren für wässrige, vor allem Borsäure / Natriumtetraborat gepufferte Zubereitungen genannt, während Ethanol, Glycerol, Ethylendiamintetraessigsäure oder auch Zitronensäure bei diesen Untersuchungen keinen bzw. sogar einen nachteiligen Effekt zeigten. Grundsätzlich wird in der einschlägigen Fachliteratur empfohlen, Epinephrin-haltige Produkte generell gekühlt aufzubewahren. Dies führt zu wesentlichen Einschränkungen bzgl. akuter Verfügbarkeitsanforderungen, insbesondere in warmen Jahreszeiten oder generell wärmeren Gegenden, besonders bei erhöhtem Allergierisiko durch Insekten, Pflanzen, Pollen u.ä.

Auch zur Inhalation durch Vernebelung vorgesehene Zubereitungen beruhen meist auf einer wässrigen, treibgasfreien Grundlage. Zur Stabilisierung wird hier insbesondere Natriumdisulfit eingesetzt, wie z. B. das früher im Handel befindliche Infectokrupp Infectopharm GmbH (enthaltend Natriumsulfit, Natriumdisulfit, Epinephrinhydrochlorid).

EP B 1051155 B1 betrifft nasal anzuwendende Zubereitungen, welche einen geeigneten Wirkstoff zur Behandlung von z. B. Sinusitis oder Rhinitis sowie Sorbit und ein wasserlösliches C₁-C₄Cellulosederivat zur Feuchthaltung der behandelten Arealen aufweisen.

US 2004/0219197 A1 beschreibt Mittel zur transdermalen Verabreichung, welche einen C₂-C₄ - Alkohol, ggf. Citratpuffer sowie zwingend einen Diethylenglykolmonoalkylether als Penetrationsverstärker aufweisen, um ausreichende Mengen Wirkstoff trotz des Fehlens bestimmter längerkettiger Fettsäuren und - Fettalkoholen bereitzustellen.

FR 2779061 offenbart ein Verfahren zur Stabilisierung von (inertgashaltigen) Zubereitungen, enthaltend phenolische Wirkstoffe, wobei Antioxidantien wie Cystein, Ascorbinsäure zusammen mit einer Poly-Hydroxysäure unter Inertgas zugesetzt werden und eine Heißsterilisation erfolgt. Ggf. können Alkohole wie z. B. Mannit als Zusatzstoffe vorhanden sein.

Gemäß der WO 98/05312 werden ethanolfreie Zubereitungen wie Sirupe u. ä. beschrieben, die einen Wirkstoff mit bitterem Geschmack und Zusätze zur Maskierung dieses bitteren Geschmacks aufweisen, wie Polyvinylpyrrolidon, sowie ein Glycyrrhizinatsalz.

Die WO 01/82931 A offenbart ein Nasenspray ohne ernsthafte Nebenwirkungen sonst üblicher adrenergisch wirksamer Mittel, wobei dennoch eine ausreichende Wirkungsmenge hiervon enthalten sein soll. Dazu werden bestimmte Mengen eines oder mehrerer adrenergisch wirksamer Verbindungen wie unter anderem Epinephrin zusammen mit dem (+)- Enantiomer von Naxolone in einem geeigneten Träger eingesetzt. WO 2004/054556 A beschreibt die einfache Herstellung poröser Partikel mit einem bestimmten Durchmesser von z.B. wenigstens 30 µm. Derartige Partikel werden erhalten durch Einsatz von Phospholipiden in organischen Lösungsmitteln, wobei nach deren Verdunsten die gewünschte Micellen - Struktur gebildet wird. Die porösen Partikel können mit Wirkstoffen beladen werden.

Die WO 99/38492 A beschreibt nasal verabreichbare Lösungen, die ein Cellulosederivat und einen Träger aus Wasser oder Wasser im Gemisch mit Propylenglykol, und/oder Glycerol aufweisen. Dadurch sollen Nebenwirkungen wie Brennen, Jucken, Reizungen der Schleimhaut vermeidbar sein.

Die EP 1 586 347 A1 und die WO 01/91848 A2 betreffen transdermale Verabreichungssysteme insbesondere zur Abgabe von Lidocain. Die diesen Wirkstoff enthaltende Zubereitung kann auch Epinephrin, EDTA, Zitronensäure und Glycerol sowie Natriummetabisulfit aufweisen.

Das Problem der Stabilisierung Epinephrin- haltiger Lösungen ist jedoch nicht angesprochen.

Bei den zur Aerosolisierung vorgesehenen Zubereitungen liegen meist nicht wässrige Grundlagen vor, da diese ein meist nicht wasserlösliches Treibgas enthalten. Da der Wirkstoff insofern in im wesentlichen nicht wässrigen Medium vorliegt, wird das Oxidationsrisiko in gewisser Weise reduziert. So beschreibt das Patent US 2868691 u.a. Epinephrin-haltige Aerosole, enthaltend den Wirkstoff, ein Lösungsmittel für Fluorchlorkohlenwasserstoffe (Treibgas) wie z. B. Chloroform, Ethanol sowie Ascorbinsäure als Stabilisator. Dabei beträgt die Menge an Treibgas in Bezug auf die Gesamtmenge der Zubereitung mindestens 50%. In der DE 699 32 835 T2 werden Aerosolzubereitungen offenbart, welche ein in Teilchenform vorliegendes Medikament wie Epinephrin, ein Treibmittel sowie einen Stabilisator, ausgewählt aus einer Aminosäure wie Glycin, Alanin, Arginin, Valin u.ä. oder Mischungen hiervon aufweisen. Auf diese Weise soll ein Absetzen des Wirkstoffs und damit eine nicht mehr kontrollier- und reproduzierbare Dosierung verhindert werden. Bei der intravenösen Verabreichung werden ebenfalls wässrige Epinephrin-haltige Lösungen verwendet, wobei Natriumdisulfit neben Schwefelsäure, Natriumchlorid eingesetzt wird, wie z. B. Adrenalin 1:1000 Jenapharm (Mibe Vertriebsgesellschaft mbH) oder Suprarenin (Sanofi- Aventis Deutschland GmbH).

### Aufgabe vorliegender Erfindung

Aufgabe vorliegender Erfindung ist es, den oben genannten Nachteilen zu begegnen und eine oral oder nasal in den Atemwegen und 1 oder in der Mundhöhle bzw. dem Rachenraum applizierbare treibgasfreie Epinephrin-haltige wässrige Zubereitung bereitzustellen, welche eine verbesserte Stabilität und somit breitere Verfügbarkeit aufweist und insofern besonders anwenderfreundlich ist.

### Lösung der Aufgabe und Gegenstand der Erfindung

Diese Aufgabe wird erfindungsgemäß dadurch gelöst, dass eine Epinephrin- oder Epinephrin-Salz, -Ester,-Hydrat- haltige wässrige Zubereitung bereitgestellt wird, die treibgasfrei ist und als Stabilisator Zitronensäure und 1 oder ein Salz hiervon oder Mischungen hiervon und / oder Ethylendiamintetraessigsäure oder ein Salz hiervon, in Kombination mit Ethanol aufweist. Insofern betrifft die vorliegende Erfindung insbesondere Zubereitungen, wobei der Stabilisator ausgewählt ist aus Zitronensäure bzw. (und / oder) einem Zitronensäuresalz in Kombination mit Ethanol. Bevorzugt kann dieser Kombination auch Ethylendiamintetraessigsäure oder ein Salz hiervon beigefügt sein. In einer weiteren Ausführungsform können Ethylendiamintetraessigsäure (EDTA) bzw. ein Salz hiervon wie das Natriumsalz (Natriumedetat) oder Mischungen hiervon und zusätzlich Ethanol hinzugesetzt werden. Die Stabilisatoren werden in geeigneten, zur Stabilisierung beitragenden Mengen wie nachfolgend angegeben eingesetzt.

Weitere geeignete Ausführungsformen gemäß vorliegender Erfindung betreffen Zubereitungen, enthaltend Zitronensäure, Ethanol sowie Ethylendiamintetraessigsäure oder ein Natriumsalz hiervon. Als Epinephrinderivat eignet sich vorzugsweise Epinephrindihydrogentartrat.

Erfindungsgemäße Zubereitungen können weiterhin Zusatzstoffe, ausgewählt aus einer oder mehreren pharmazeutisch verträglichen Säuren (beispielsweise Schwefelsäure, Salzsäure, Ascorbinsäure), Salze wie z. B. die unten genannten Elektrolyte oder andere dem Fachmann bekannte Salze, Konservierungsmittel(n), pH-Wert-Regulatoren, Propylenglykol, oder Mischungen hiervon enthalten.

Die Zubereitungen liegen vorzugsweise in gebrauchsfertiger Form, z. B. geeigneten Flaschen oder Ampullen vor und weisen insbesondere eine Osmolarität von 200- 350 mOsmol/ kg auf. Der pH-Wert der gebrauchsfertigen Zubereitungen beträgt vorzugsweise 2,0-3,3. Die Zubereitungen können auch in Kombination mit einer elektrischen oder mechanischen (Pumpzerstäuber) Verneblungs- oder Zerstäubungsvorrichtung (Spray) zur Verwendung, vor allem, bei akuten oder chronischen allergischen Reaktionen, insbesondere der Atemwege wie nachfolgend beschrieben, vorliegen.

Überraschender Weise kann durch diese Stabilisatoren die Haltbarkeit Epinephrin-haltiger wässriger Zubereitungen derart verbessert werden, dass eine ständige Aufbewahrung unter Kühlung nicht mehr erforderlich ist. Somit können risikobehaftete Personen insbesondere auch im Sommer oder in generell wärmeren Gegenden sehr viel einfacher versorgt werden, da diese die erfindungsgemäße Zubereitung z.B. als Spray bei sich tragen (also auch ohne Kühlung transportieren) können und im Bedarfsfall, z. B. in akuten Situationen wie Insektenstich u.ä. jederzeit versorgt werden können. Darüber hinaus kann durch die erhöhte Stabilität eine verbesserte (kontrollierte) Verabreichung des aktiven Wirkstoffes erfolgen. Dies war insbesondere deshalb nicht zu erwarten, da bereits ähnliche Zusätze gemäß Stand der Technik wie oben genannt eingesetzt wurden, ohne dass eine entsprechende Effizienz festgestellt bzw. in vielen Fällen sogar ein deutlich negativer Einfluss der Einzelkomponenten auf die Stabilität des Epinephrins festgestellt wurde.

### Nähere Erläuterung der Erfindung, bevorzugte Ausführungsformen

### 1. Inhaltsstoffe

Als Wirkstoff in erfindungsgemäßen Zubereitungen wird Epinephrin oder ein pharmazeutisch verträgliches (Epinephrin-) Salz, Ester, Hydrat eingesetzt. Insbesondere bevorzugt sind Salze wie Hydrochlorid oder Dihydrogentartrat. Besonders bevorzugt ist Epinephrindihydrogentartrat. Der Wirkstoff liegt gewöhnlich in pharmazeutisch zulässigen Mengen wie 0,1 bis 12 Gew.%, vorzugsweise 0,2 bis 5 Gew.%, bezogen auf die Gesamtmenge der Zubereitung vor, entsprechend etwa 0,05 bis 10 % Wirkstoffbase, sofern Derivate verwendet werden.

Als Stabilisator wird Zitronensäure oder ein Salz hiervon, z. B. Natriumcitrat oder Mischungen hiervon in Kombinationen wie angegeben eingesetzt. Dies hat den Vorteil einer olfaktorisch verbesserten Anwendungsform, da die applizierte Lösung als geschmacklich sehr angenehm wahrgenommen wird. Dieser Stabilisator kann in einer der Gesamtzubereitung angemessenen Menge, insbesondere in einer Menge von 0,05 bis 2 Gew.%, insbesondere 0,5 bis 1,5 Gew.%, vor allem 0,5 bis 1 Gew.%, eingesetzt werden.

Diese Angabe bezieht sich auf die Menge an Zitronensäure oder Zitronensäuresalz oder Gemischen hiervon. Unter Zitronensäuresalz wird gemäß vorliegender Erfindung ein Salz hiervon, wie z. B. Natriumcitrat verstanden. Vorzugsweise wird Zitronensäure eingesetzt.

Es ist erfindungsgemäß insofern möglich, insbesondere auf bisher eingesetzte Puffer wie vor allem aus Borsäure / Natriumtetraborat zu verzichten. Die erfindungsgemäßen Zubereitungen sind daher vorzugsweise frei von Borsäure / Natriumtetraborat (Puffer).

Ethanol eignet sich insbesondere in einer Menge von 0,5 bis 25 Gew. %, bevorzugt 1 bis 20 Gew.%, vor allem 1 bis 10 Gew.%. Besonders bevorzugt ist Ethanol in bevorzugten bzw. besonders bevorzugten Mengen wie vor allem 0,5 bis 15 Gew. %, vorzugsweise auch 1 bis 10 Gew.% oder auch 0,5 bis 8 Gew.%.

Ein weiterer geeigneter Stabilisator als Teil einer erfindungsgemäßen Stabilisatorkombination in oben genannten Zubereitungen ist Ethylendiamintetraessigsäure (EDTA), vor allem als Natriumsalz, wie Natriumedetat. Dieses kann vorzugsweise in Mengen von 0,005 bis 1 Gew.% vorliegen. Vorzugsweise beträgt die Menge 0,01 bis 0,1 Gew.%.

Alle Mengenangaben beziehen sich auf die gebrauchsfertige zur oralen oder nasalen Anwendung direkt einsetzbare Lösung. Diese weist vorzugsweise eine Osmolarität von 200-350, vor allem 250-350 mOsmol / kg auf. Höher osmolare Lösungen können durch Verdünnung, nieder osmolare Lösungen durch Zugabe inerter Zusatzstoffe wie nachfolgend beschrieben auf die gewünschten Werte eingestellt werden. Ebenso können erfindungsgemäße Epinephrin oder Epinephrinderivate haltige wässrige Lösungen in Bezug auf die Inhaltsstoffe konzentrierter hergestellt werden, so dass durch Verdünnung mit Lösungsmittel, insbesondere Wasser, die oben genannten Anwendungskonzentrationen erhalten werden. Beispielsweise können Ausgangszubereitungen bis zu 50% Ethanol oder bis zu 5 % Zitronensäure oder bis zu 1,5 % EDTA aufweisen.

Die erfindungsgemäße Zubereitung weist wie erläutert Wasser (vorzugsweise für pharmakologische Zwecke gereinigtes Wasser) im gebrauchsfertigen Produkt in einer Menge von ca. 65- 98 Gew.% auf.

Besonders bevorzugt sind Zubereitungen, enthaltend Zitronensäure, z. B. in einer Menge von 0,05 bis 1 Gew.%.

Eine weitere bevorzugte Ausführungsform umfasst neben Zitronensäure, z. B. 0,05 bis 1,5 Gew. %, wie beschrieben, 0,5 bis 15 Gew.% Ethanol, vorzugsweise 0,5 bis 10 Gew.% Ethanol wie genannt oder in bevorzugten Mengen wie oben angegeben. Es ist auch bevorzugt, Natriumedetat in Kombination mit Zitronensäure und Ethanol vor allem in den jeweils angegebenen Mengen (bezogen auf die gebrauchsfertige Zubereitung) einzusetzen.

### 2. Zusatzstoffe

Es hat sich gezeigt, dass der pH- Wert der gebrauchsfertigen Lösungen vorzugsweise zwischen 2 und 4,3, bevorzugt zwischen 2 und 3,8, vor allem 2,0 bis 3,3, insbesondere 2,3 bis 3,2 liegt. Zur genauen Einstellung auf gewünschte Werte können dem Fachmann hierfür bekannte Mittel (pH-Wert- Regulatoren) wie beispielsweise Ascorbinsäure oder ein Salz hiervon, z. B. Natriumascorbat, Säuren wie Salzsäure, Schwefelsäure, Basen wie NaOH oder andere geeignete pH- Wert-Regulatoren wie Citrate eingesetzt werden.

Zur Konservierung empfiehlt sich der Zusatz hierfür geeigneter Mittel wie insbesondere Benzoesäure oder Chlorbutanol in dem Fachmann geeigneten Mengen.

Wie oben erläutert, kann die Osmolarität einerseits durch Verdünnen mit Lösungsmittel oder andererseits durch Zusatz geeigneter Hilfsmittel wie z. B. Propylenglykol, z. B. bis 2% je nach gewünschter Herstellungsform eingestellt werden. Erfindungsgemäße Zubereitungen enthalten vorzugsweise Propylenglykol, z. B. in Mengen von 0,01 bis 1,5 Gew. %, vor allem 0,1 bis 1 Gew. %.

Weitere dem Fachmann für derartige Zubereitungen bekannte Zusatzstoffe können beispielsweise gewählt werden aus Elektrolyten wie Natriumchlorid, Kaliumchlorid, Magnesiumsulfat, Kalziumchlorid, oder auch 1,2- Propandiol oder ggf. Natriumdisulfit oder Mischungen hiervon. Bevorzugt werden Elektrolyte, 1,2 Propandiol oder Mischungen hiervon gewählt. In einer weiteren Ausführungsform können weitere Zusatzstoffe wie genannt, bevorzugt jedoch nicht Natriumdisulfit enthalten sein, so dass die Zubereitung insofern in einer Ausführungsform auch frei hiervon sein kann.

Die vorliegenden Zubereitungen weisen bevorzugt den genannten Wirkstoff, den genannten Alkohol, (nicht jedoch mehr als 3-wertige Alkohole wie Sorbit, Mannit), ferner stabilisierende Zusätze und die oben genannten Zusatzstoffe auf. Dabei sind bei den Säure-Zusatzstoffen Ascorbinsäure- oder Derivate hiervon wie Salze und ähnliche bevorzugt überwiegend nicht erforderlich. Auch bedarf es hierbei (bevorzugt) keiner Penetrationsverstärker wie Diethylenglykolmonoalkylether, PVP oder Süßstoffen wie Glycyrrethinatsalzen. Erfindungsgemäß ist daher ein Minimum an Zusatzstoffen möglich, was weiterhin zur Stabilisierung - auch ohne Inertgas bei der Herstellung - beiträgt.

Die vorliegende Erfindung betrifft insofern insbesondere Epinephrin- oder ein Epinephrin- Salz, -Ester, -Hydrat - enthaltende wässrige treibgasfreie Zubereitungen, vor allem zur oralen, vor allem inhalativ- oralen, Anwendung bei akuten oder chronischen allergischen Reaktionen; vor allem aber auch bei allergischem Schock, allergiebedingter Atemnot, allergisch bedingter Verengung der Luftwege, oder Herz-Kreislauf-Stillstand, bzw. obstruktiver Bronchitis des Säuglings und Kleinkindes, Asthma; oder aber auch bei Pseudokrupp, Säuglings-Bronchiolitis, oder sonstigen Formen der Atemnot, welche dadurch gekennzeichnet sind, dass sie zusammengesetzt sind aus Epinephrin, oder einem Epinephrin- Salz, -Ester, - Hydrat, Wasser, einem stabilisierenden Zusatz, ausgewählt aus Zitronensäure 1 oder einem Zitronensäuresalz und / oder Ethylendiamintetraessigsäure oder ein Salz hiervon in Kombination mit Ethanol sowie einem oder mehreren Zusatzstoffen, ausgewählt aus einer oder mehreren pharmazeutisch verträglichen Säuren, Salzen, Konservierungsmitteln, pH- Wert- Regulatoren, Propylenglykol oder Mischungen hiervon.

Bevorzugt ist die Zusammensetzung bereitet wie oben beschrieben mit den vor allem geeigneten Inhaltsstoffen.

### Herstellung

Erfindungsgemäße Zubereitungen werden hergestellt, indem man Wasser, eventuell gewünschte Zusatzstoffe sowie Wirkstoff und Stabilisatoren in beliebiger Reihenfolge z. B. bei Raumtemperatur, vorzugsweise unter Inertgasatmosphäre (wie z. B. Stickstoff) mischt und sodann in geeignete Gefäße, vorzugsweise Glasbehälter oder Ampullen (z. B. Braunglas oder Weißglas), insbesondere unter Sauerstoffausschluss, füllt und diese mit geeigneten Verschlüssen versieht. Die Aufbewahrung erfolgt vorzugsweise unter Lichtausschluss, z. B. bei Raumtemperatur.

### Anwendung

Die Zubereitungen gemäß vorliegender Erfindung können ohne Kühlung, vor allem über längere z. B. Transport-Zeiträume, gelagert werden und sind insofern flexibel einsetzbar. Sie können mittels geeigneter Vernebelungs- oder Zerstäubungsvorrichtungen oder anderen Applikationssystemen dem Atemwegstrakt oral oder auch nasal, insbesondere der Mundhöhle, dem Rachenraum oder den Atemwegen zugeführt werden, um eine allergisch bedingte akute oder chronische Reaktion zu bekämpfen, z. B. durch Bronchialdilatation und/oder Vasokonstriktion. Dazu gehören vor allem akute oder chronische allergische Reaktionen wie allergischer Schock, allergiebedingte Atemnot, allergisch bedingte Verengung der Luftwege; ferner Herz-Kreislauf-Stillstand bzw. obstruktive Bronchitis des Säuglings und Kleinkindes, Asthma, oder auch Pseudokrupp, Säuglingsbronchiolitis, oder sonstige Formen der Atemnot. Die Anwendung kann insofern insbesondere durch Inhalation (nasal / oral) oder auch durch Zerstäuben / Versprühen (oral / nasal) oder durch Instillation erfolgen. Zur Vernebelung eignen sich bekannte Systeme wie elektrische Vernebler oder mechanische Systeme wie Pumpzerstäuber mit Sprühkopf. Elektrische Systeme können für den Anwendungsfall durch Entfernung der Verschlusskappe des die Zubereitung enthaltenden Gefäßes wie Glasflasche und Aufsetzen einer Dosierpumpe mit Tropfapplikator befüllt werden. Als elektrische Vernebelungssysteme eigenen sich insbesondere hierfür bekannte Düsenvernebler wie z.B. PariBoy SX®/ PARI LC Sprint®, Pari Sinus® oder Pari TurboBoy N® /LC Plus®, Vibrationsvernebler wie z.B. PARI eFlow Rapid® oder Ultraschallvernebler wie z.B. I-neb AAD®. Derartige Vernebler (Dosiervorrichtungen) sind bekannt und beispielsweise beschrieben in EP 1 847 256 A1. Zur Zerstäubung sind beispielsweise Standard - Zerstäuberpumpen der Firmen Pfeiffer und Aeropump geeignet. Insofern ist es möglich, erfindungsgemäße Zubereitungen kontrolliert dosiert anwendungssicher zu vernebeln oder zu zerstäuben, um sie nasal oder oral sicher zum gewünschten Wirkort zu bringen. Dies ist insbesondere im Akutfall wesentlich, um Folgen allergischer Überreaktionen wie Schock, Atemnot, Verengung der Atemwege bis hin zu Ersticken zu verhindern, mindestens jedoch zu bekämpfen. Da die erfindungsgemäßen Zubereitungen nicht (ständig) gekühlt aufbewahrt werden müssen, sind sie somit für Risikopersonen sehr viel leichter verfügbar und einsetzbar.

Die erfindungsgemäßen Zubereitungen bzw. verwendungsgemäß hergestellten Epinephrinzubereitungsmittel wie beschrieben dienen insofern einerseits der Prävention, andererseits aber der wirksamen Behandlung beschriebener Zustände.

### Beispiele

Die Erfindung wird anhand nachfolgender Beispiele näher erläutert.

### Beispiele 1 - 12

### A. Beschreibung der Zubereitungen

Folgende Zubereitungen wurden wie beschrieben hergestellt (ohne Anwendung von Inertgas). Zunächst wurde eine Grundformulierung bereitet, umfassend 7,29 g Epinephrindihydrogentartrat, 5 g Propylenglycol, 1 g Benzoesäure, 1 g Natriumchlorid. Dieser wurden dann die in der Tabelle 1 unten angegebenen Stabilisatoren hinzugefügt und auf 1000 ml mit Wasser aufgefüllt. Die erhaltenen Zubereitungen gemäß den Beispielen 1-6 sind Vergleichsprodukte, Beispiele 7-12 sind erfindungsgemäße Zubereitungen. Die Produkte wurden nach der Herstellung in Glasflaschen gefüllt und verschlossen.

**Tabelle 1**

| Beispiel Nr. | Alkoholischer Stabilisator | Natriumedetat [g] | Zitronensäure [g] |
|---|---|---|---|
| 1 | - | 0,15 | - |
| 2 | - | - | - |
| 3 | 6 % Ethanol | - | - |
| 4 | 12 % Ethanol | - | - |
| 5 | 24 % Ethanol | - | - |
| 6 | 6 % Glycerol | - | - |
| 7 | 12 % Ethanol | 0,15 | - |
| 8 | 24 % Ethanol | 0,15 | - |
| 9 | 6 % Glycerol | 0,15 | |
| 10 | 6 % Ethanol | - | 12 |
| 11 | 6 % Glycerol | - | 12 |
| 12 | 6 % Diethylenglykol | - | 12 |

### B. Stabilitätstests

Wie erläutert, unterliegen Epinephrin-haltige Zubereitungen der Zersetzung, was zu farbigen Abbauprodukten (Melanin-Komponenten) führt. Deren Entstehen bzw. Vorhandensein lässt sich anhand von Extinktionsmessungen (Ermittlung der Farbe der Zubereitung) bestimmen und insofern die Stabilität einer Lösung ermitteln. Dazu wurden die Extinktionswerte oben beschriebener Zubereitungen zu Beginn des Tests (t=0), nach einer Lagerzeit von 1 Woche bei 40° C / 75% rel. Luftfeuchtigkeit (t=1 W) und nach einer Lagerzeit von 1 Monat bei 25° C / 60% rel. Luftfeuchtigkeit (t= 1 M) mittels UV- Detektion ermittelt. Dabei wurden die in Tabelle 2 angegebenen Ergebnisse erhalten:

**Tabelle 2**

| Zubereitung | Extinktionswerte t=0 | Extinktionswerte t=1 W (40°C) | Extinktionswerte t=1 M (25°C) |
|---|---|---|---|
| Erfindung (7-12) | 0,0000 - 0,0070 | 0,0075 - 0,0809 | 0,0125 - 0,0715 |
| Vergleich (1-6) | 0,0005 - 0,0722 | 0,1135 - 0,4202 | 0,0855 - 0,3967 |

### Beispiele 13 - 22

### A. Beschreibung der Zubereitungen

Folgende Zubereitungen wurden wie beschrieben hergestellt (ohne Anwendung von Inertgas). Zunächst wurde eine Grundformulierung bereitet aus Epinephrindihydrogentartrat (7,29 g), Benzoesäure (1,0 g), Wasser (ad 1000 ml), wobei die in Tabelle 3 angegebenen Stabilisatoren hinzugesetzt wurden. Die erhaltenen Zubereitungen gemäß den Beispielen 13-22 sind erfindungsgemäße Zubereitungen. Die Produkte wurden nach der Herstellung in Glasflaschen gefüllt und verschlossen.

**Tabelle 3**

| Beispiel Nr. | Ethanol [g] | Glycerol [g] | Propylene glycol [g] | Natriumedetate [g] | Zitronensäure [g] |
|---|---|---|---|---|---|
| 13 | 8 | - | - | 0,1 | 12 |
| 14 | 8 | - | - | 0,3 | 12 |
| 15 | 140 | - | 8 | 0,1 | 12 |
| 16 | - | 8 | - | 0,1 | 12 |
| 17 | - | 120 | 8 | 0,1 | 12 |
| 18 | 12 | - | - | 0,1 | 6 |
| 19 | 12 | - | - | 0,3 | 6 |
| 20 | 60 | - | - | 0,1 | 6 |
| 21 | - | 60 | - | 0,1 | 6 |
| 22 | - | 12 | 6 | 0,3 | 6 |

### B. Stabilitätstests:

Wie erläutert, kann anhand der oben beschriebenen Extinktionsmessungen (Farb-Ermittlung) der Zubereitungen deren Zersetzungsgrad bestimmt und insofern die Stabilität einer Lösung ermittelt werden. Dazu wurden die Extinktionswerte oben beschriebener Zubereitungen 13-22 zu Beginn des Tests (t=0), nach einer Lagerzeit von 1 Monat bei 40° C / 75% rel. Luftfeuchtigkeit (t=1 M) und nach einer Lagerzeit von 3 Monaten bei 25° C / 60% rel. Luftfeuchtigkeit (t= 3 M) mittels UV- Detektion ermittelt. Dabei wurden die in Tabelle 4 angegebenen Ergebnisse erhalten:

**Tabelle 4**

| Zubereitung | Extinktionswerte t=0 | Extinktionswerte t=1 M (40°C) | Extinktionswerte t=3 M (25°C) |
|---|---|---|---|
| Erfindung (13-22) | 0,0009 - 0,0090 | 0,0743 - 0,3170 | 0,0343 - 0,1466 |

## Patentansprüche

1. Eine Epinephrin- oder ein Epinephrin-Salz, -Ester, -Hydrat enthaltende wässrige treibgasfreie Zubereitung, **dadurch gekennzeichnet, dass** sie einen stabilisierenden Zusatz, ausgewählt aus Zitronensäure und/oder Zitronensäuresalz und/oder Ethylendiamintetraessigsäure oder ein Salz hiervon in Kombination mit Ethanol aufweist.

2. Zubereitung gemäß Anspruch 1, **dadurch gekennzeichnet, dass** sie als stabilisierenden Zusatz Zitronensäure und/oder ein Zitronensäuresalz und als Stabilisator Ethanol aufweist.

3. Zubereitung gemäß Anspruch 2, **dadurch gekennzeichnet, dass** sie weiterhin Ethylendiamintetraessigsäure oder ein Salz hiervon aufweist.

4. Zubereitung gemäß Anspruch 1, **dadurch gekennzeichnet, dass** sie als stabilisierenden Zusatz Ethylendiamintetraessigsäure oder ein Salz hiervon in Kombination mit Ethanol aufweist.

5. Zubereitung gemäß einem der Ansprüche 1 bis 4, **dadurch gekennzeichnet, dass** weiterhin ein oder mehrere Zusatzstoffe, ausgewählt aus einer oder mehreren pharmazeutisch verträglichen Säuren, Salzen, Konservierungsmitteln, pH- Wert-Regulatoren, Propylenglykol oder Mischungen hiervon, enthalten sind.

6. Zubereitung gemäß einem der Ansprüche 1 bis 5, **dadurch gekennzeichnet, dass** sie in gebrauchsfertiger Form vorliegt und eine Osmolarität von 200 - 350 mOsmol/kgaufweist.

7. Zubereitung gemäß einem der Ansprüche 1 bis 6 , **dadurch gekennzeichnet, dass** sie Epinephrindihydrogentartrat aufweist.

8. Eine Epinephrin- oder ein Epinephrin- Salz, -Ester, -Hydrat - enthaltende wässrige treibgasfreie Zubereitung gemäß einem der Ansprüche 1 bis 7, **dadurch gekennzeichnet, dass** sie zusammengesetzt ist aus Epinephrin, oder einem Epinephrin- Salz, -Ester, -Hydrat, Wasser, einem stabilisierenden Zusatz, ausgewählt aus Zitronensäure/oder einem Zitronensäuresalz und/oder Ethylendiamintetraessigsäure oder einem Salz hiervon, in Kombination mit Ethanol sowie einem oder mehreren Zusatzstoffen, ausgewählt aus einer oder mehreren pharmazeutisch verträglichen Säuren, Salzen, Konservierungsmitteln, pH- Wert- Regulatoren, Propylenglykol oder Mischungen hiervon.

9. Zubereitung gemäß einem der Ansprüche 1 bis 8 in Kombination mit einer Vernebelungs- oder Zerstäubungsvorrichtung oder einem anderen Applikations-system.

10. Zubereitung gemäß einem der Ansprüche 1 bis 8 zur Anwendung bei allergischem Schock, allergiebedingter Atemnot, allergisch bedingter Verengung der Luftwege oder Herz-Kreislauf-Stillstand bzw. obstruktiver Bronchitis des Säuglings und Kleinkindes, Asthma, Pseudokrupp, Säuglingsbronchiolitis oder sonstigen Formen der Atemnot.

11. Zubereitung gemäß einem der Ansprüche 1 bis 8 zur oralen oder nasalen Anwendung bei allergischem Schock, allergiebedingter Atemnot, allergisch bedingter Verengung der Luftwege oder Herz-Kreislauf-Stillstand, bzw. obstruktiver Bronchitis des Säuglings und Kleinkindes, Asthma, Pseudokrupp, Säuglingsbronchiolitis, oder sonstigen Formen der Atemnot.

12. Zubereitung gemäß einem der Ansprüche 1-8 zur therapeutischen oralen oder nasalen Anwendung durch Inhalation, Zerstäubung/Versprühen oder Instillation.

13. Verfahren zur Stabilisierung einer wässrigen Epinephrin- oder ein Epinephrin-Salz, -Ester, -Hydrat - enthaltenden Zubereitung gemäß einem der Ansprüche 1 bis 8, **dadurch gekennzeichnet, dass** man der wässrigen Epinephrin- oder ein Epinephrin- Salz, -Ester, -Hydrat- enthaltenden Zubereitung Zusätze, ausgewählt aus Zitronensäure/Zitronensäuresalz und/oder Ethylendiamintetraessigsäure oder einem Salz hiervon in Kombination mit Ethanol hinzufügt.

14. Verwendung einer Zubereitung gemäß einem der Ansprüche 1 bis 8 zur Herstellung eines Mittels zur oralen oder nasalen therapeutischen Anwendung in der Mundhöhle, dem Rachenraum oder den Atemwegen.

## Claims

1. An aqueous, propellant gas-free preparation containing epinephrine or an epinephrine salt, ester or hydrate, **characterised in that** it comprises a stabilising addition selected from citric acid and/or citric acid salt and/or ethylenediaminetetraacetic acid or a salt thereof, in combination with ethanol.

2. A preparation according to claim 1, **characterised in that** it comprises citric acid and/or a citric acid salt as stabilising addition and ethanol as stabiliser.

3. A preparation according to claim 2, **characterised in that** it furthermore comprises ethylenediaminetetraacetic acid or a salt thereof.

4. A preparation according to claim 1, **characterised in that** it comprises ethylenediaminetetraacetic acid or a salt thereof in combination with ethanol as stabilising addition.

5. A preparation according to one of claims 1 to 4, **characterised in that** it furthermore contains one or more additives selected from one or more pharmaceutically acceptable acids, salts, preservatives, pH regulators, propylene glycol or mixtures thereof.

6. A preparation according to one of claims 1 to 5, **characterised in that** it is in ready-to-use form and has an osmolarity of 200-350 mOsmol/kg.

7. A preparation according to one of claims 1 to 6, **characterised in that** it comprises epinephrine bitartrate.

8. An aqueous, propellant gas-free preparation containing epinephrine or an epinephrine salt, ester or hydrate according to one of claims 1 to 7, **characterised in that** it is composed of epinephrine, or an epinephrine salt, ester or hydrate, water, a stabilising addition selected from citric acid/or a citric acid salt and/or ethylenediaminetetraacetic acid or a salt thereof, in combination with ethanol and one or more additives selected from one or more pharmaceutically acceptable acids, salts, preservatives, pH regulators, propylene glycol or mixtures thereof.

9. A preparation according to one of claims 1 to 8 in combination with a nebulising or atomising device or another administration system.

10. A preparation according to one of claims 1 to 8 for application in anaphylactic shock, allergic dyspnoea, allergic constriction of the airways or cardiac arrest, or obstructive infant and newborn bronchitis, asthma, pseudocroup, infant bronchiolitis or other forms of dyspnoea.

11. A preparation according to one of claims 1 to 8 for oral or nasal application in anaphylactic shock, allergic dyspnoea, allergic constriction of the airways or cardiac arrest, or obstructive infant and newborn bronchitis, asthma, pseudocroup, infant bronchiolitis or other forms of dyspnoea.

12. A preparation according to one of claims 1 to 8 for therapeutic oral or nasal application by inhalation, atomisation/spraying or instillation.

13. A method for stabilising an aqueous preparation containing epinephrine or an epinephrine salt, ester or hydrate according to one of claims 1 to 8, **characterised in that** additions selected from citric acid/citric acid salt and/or ethylenediaminetetraacetic acid or a salt thereof, in combination with ethanol, are added to the aqueous preparation containing epinephrine or an epinephrine salt, ester or hydrate.

14. Use of a preparation according to one of claims 1 to 8 for producing an agent for oral or nasal therapeutic application in the oral cavity, pharynx or airways.

## Revendications

1. Préparation aqueuse exempte de gaz propulseur contenant de l'épinéphrine ou un sel, un ester, un hydrate d'épinéphrine, **caractérisée en ce qu'**elle comprend un additif de stabilisation choisi parmi l'acide citrique et/ou un sel d'acide citrique et/ou l'acide éthylènediaminetétraacétique ou un de ses sels, en combinaison avec de l'éthanol.

2. Préparation selon la revendication 1, **caractérisée en ce qu'**elle comprend, à titre d'additif de stabilisation, de l'acide citrique et/ou un sel d'acide citrique et, à titre de stabilisateur, de l'éthanol.

3. Préparation selon la revendication 2, **caractérisée en ce qu'**elle comprend en outre de l'acide éthylènediaminetétraacétique ou un de ses sels.

4. Préparation selon la revendication 1, **caractérisée en ce qu'**elle comprend, à titre d'additif de stabilisation, de l'acide éthylènediaminetétraacétique ou un de ses sels en combinaison avec de l'éthanol.

5. Préparation selon l'une quelconque des revendications 1 à 4, **caractérisée en ce qu'**elle contient en outre un ou plusieurs additifs pharmaceutiquement acceptables choisis parmi le groupe comprenant des acides, des sels, des conservateurs, des régulateurs de la valeur du pH, du propylèneglycol, ou leurs mélanges.

6. Préparation selon l'une quelconque des revendications 1 à 5, **caractérisée en ce qu'**elle se présente sous une forme prête à l'emploi et qu'elle comprend une osmolarité de 200 à 350 mOsmol/kg.

7. Préparation selon l'une quelconque des revendications 1 à 6, **caractérisée en ce qu'**elle comprend de l'hydrogénotartrate d'épinéphrine.

8. Préparation aqueuse exempte de gaz propulseur contenant de l'épinéphrine ou un sel, un ester, un hydrate d'épinéphrine selon l'une quelconque des revendications 1 à 7, **caractérisée en ce qu'**elle se compose d'épinéphrine ou d'un sel, d'un ester, d'un hydrate d'épinéphrine, d'eau, d'un additif de stabilisation choisi parmi l'acide citrique ou un sel d'acide citrique et/ou l'acide éthylènediaminetétraacétique ou un de ses sels, en combinaison avec de l'éthanol, et d'un ou de plusieurs additifs pharmaceutiquement acceptables choisis parmi le groupe comprenant des acides, des sels, des conservateurs, des régulateurs de la valeur du pH, du propylèneglycol, ou leurs mélanges.

9. Préparation selon l'une quelconque des revendications 1 à 8, en combinaison avec un dispositif de nébulisation ou de pulvérisation ou avec un autre système d'application.

10. Préparation selon l'une quelconque des revendications 1 à 8, pour son application en cas de choc allergique, d'une dépression respiratoire d'origine allergique, d'un rétrécissement des voies respiratoires dû à une allergie ou d'un arrêt cardiovasculaire, respectivement, de bronchite obstructive du nourrisson et de l'enfant en bas âge, d'asthme, de laryngite striduleuse, de bronchiolite du nourrisson ou d'autres formes de dépression respiratoire.

11. Préparation selon l'une quelconque des revendications 1 à 8, pour son application par voie orale ou par voie nasale en cas de choc allergique, d'une dépression respiratoire d'origine allergique, d'un rétrécissement des voies respiratoires dû à une allergie ou d'un arrêt cardiovasculaire, respectivement, de bronchite obstructive du nourrisson et de l'enfant en bas âge, d'asthme, de laryngite striduleuse, de bronchiolite du nourrisson ou d'autres formes de dépression respiratoire.

12. Préparation selon l'une quelconque des revendications 1 à 8, pour son application thérapeutique par voie orale ou par voie nasale par inhalation, pulvérisation/nébulisation ou instillation.

13. Procédé pour la stabilisation d'une préparation aqueuse contenant de l'épinéphrine ou un sel, un ester, un hydrate d'épinéphrine selon l'une quelconque des revendications 1 à 8, **caractérisé en ce qu'**on ajoute à la préparation aqueuse contenant de l'épinéphrine ou un sel, un ester, un hydrate d'épinéphrine, des additifs choisis parmi l'acide citrique / un sel d'acide citrique et/ou l'acide éthylènediaminetétraacétique ou un de ses sels, en combinaison avec de l'éthanol.

14. Utilisation d'une préparation selon l'une quelconque des revendications 1 à 8, pour la production d'un agent destiné à une application thérapeutique par voie orale ou par voie nasale dans la cavité buccale, dans le pharynx ou dans les voies respiratoires.
